(19) Europäisches Patentamt
European Patent Office
Office européen des brevets

(11) Publication number: 0 140 589 B1

(12) EUROPEAN PATENT SPECIFICATION

(45) Date of publication of patent specification: 25.05.88

(51) Int. Cl.[4]: C 12 Q 1/32, G 01 N 21/75, G 01 N 33/64

(21) Application number: 84306671.3

(22) Date of filing: 28.09.84

# (54) Enzymatic determination of D-3-hydroxybutyric acid or acetoacetic acid, and reagents therefor.

(30) Priority: 27.10.83 JP 199928/83

(43) Date of publication of application: 08.05.85 Bulletin 85/19

(45) Publication of the grant of the patent: 25.05.88 Bulletin 88/21

(84) Designated Contracting States: CH DE FR GB IT LI

(56) References cited:
DE-A-2 929 088
DE-B-2 518 862
PATENT ABSTRACTS OF JAPAN, unexamined applications, P field, vol.4, no.66, May 17, 1980 THE PATENT OFFICE JAPANESE GOVERNMENT, page 145P11
PATENT ABSTRACTS OF JAPAN, unexamined applications, P filed, vol.8, no.92, April 27, 1984 THE PATENT OFFICE JAPANESE GOVERNMENT, page 48P271

**The file contains technical information submitted after the application was filed and not included in this specification**

(73) Proprietor: SANWA KAGAKU KENKYUSHO CO., LTD.
No. 35, Higashi-sotobori-cho
Higashi-ku Nagoya-shi Aichi-ken (JP)

(72) Inventor: Shigeta, Yukio
6-3, Minatogawa-cho
Hyogo-ku Kobe-shi Hyogo-ken (JP)
Inventor: Harano, Yutaka
11-100 Aza-Nakayamadani
Tanakami-inazu-cho Otsu-shi Shiga-ken (JP)
Inventor: Kurono, Masayasu
2-72-2 Kakeago-cho
Minami-ku Nagoya-shi Aichi-ken (JP)
Inventor: Awaya, Juichi
1-111 Amakoda Moriyama-shi
Nagoya-shi Aichi-ken (JP)
Inventor: Uno, Shizuo
2-116-2 Fujigaoka
Kasugai-shi Aichi-ken (JP)

(74) Representative: Diamond, Bryan Clive et al
Gee & Co. Chancery House Chancery Lane
London WC2A 1QU (GB)

EP 0 140 589 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an easy method for the diagnosis of diabetes and a reagent for carrying out the method. According to the invention, a mammalian, especially human body fluid such as blood serum and plasma, or urine is employed as a sample therefor.

It has been known that an insufficient supply of carbohydrates, insufficient utilization thereof, stress, physical exercise, diabetes and the like cause an acceleration of s decomposition of lipids and an oxidation of fatty acids to increase concentrations of D-3-hydroxybutyric acid and acetoacetic acid in blood and urine. Measurement of such compounds has great significance in giving a usable index to determine the progress of diabetes.

The following three methods for determining D-3-hydroxybutyric acid or acetoacetic acid have been previously proposed by the co-inventors of the present invention:

(a) A quantitative determination of acetoacetic acid in blood, wherein serum acetoacetic acid is reacted with *p*-nitrophenyldiazonium fluoborate to form a hydrazo-compound, a solution of which is subjected to colorimetry (Jap. Pat. Appln. No. 86803/1978, published in Jap. Unexamined Pat. Appln. No. 15004/1980);

(b) A quantitative determination of D-3-hydroxybutyric acid in blood, wherein serum D-3-hydroxybutyric acid is converted to acetoacetic acid through an enzymatic reaction, the resulting acetoacetic acid and acetoacetic acid present previously in the blood serum are converted into a hydrazo-compound, a solution containing the hydrazo-compound is subjected to colorimetry, as in the method (a) to determine the concentration of the total acetoacetic acid, the concentration of the acetoacetic acid converted from D-3-hydroxybutyric acid being calculated by deducting the concentration of the acetoacetic acid present previously in the blood serum from the total acetoacetic acid concentration to determine a concentration of serum D-3-hydroxybutyric acid (Jap. Pat. Appln. No. 108 153/1978, published in Jap. Unexamined Pat. Appln. Gazette No. 35 232/1980); and

(c) A quantitative determination of D-3-hydroxybutyric acid in blood, wherein serum D-3-hydroxybutyric acid is converted to acetoacetic acid through an enzymatic reaction the resulting acetoacetic acid and *p*-nitrophenyldiazonium fluoborate are reacted in the presence of a surface active agent, and an alkali hydroxide is added to the resulting reaction mixture to form a stable azo-compound through a hydrazo-compound, a solution of said azo-compound being subjected to a colorimetry as in the method (a), as well as reagents therefor (Jap. Pat. Appln. No. 112 520/1982, published in Jap. Unexamined Pat. Appln. Gazette No. 5 959/1984).

Each of said methods shows a high sensitivity and accuracy, but the methods (a) to (c) have a disadvantage of requiring a pretreatment of deproteinization or dialysis of the sample.

An enzymatic quantitative determination of D-3-hydroxybutyric acid or acetoacetic acid, according to the invention utilizes a reversible reaction as shown below:

$$\text{D-3-OHBA} + \text{NAD}^+ \underset{}{\overset{\text{3-OHBDH}}{\rightleftarrows}} \text{AcAc} + \text{NADH} + \text{H}^+$$

wherein,

D-3-OHBA: D-3-hydroxybutyric acid,
NAD: Nicotinamide adenine dinucleotide,
3-OHBDH: 3-hydroxybutyric dehydrogenase,
AcAc: Acetoacetic acid, and
NADH: Reduced nicotinamide adenine dinucleotide.

As is seen from the reaction equation, D-3-hydroxybutyric acid (D-3-OHBA) in a body fluid or urine is reacted in the presence of 3-hydroxybutyric dehydrogenase (3-OHBDH) with nicotinamide adenine dinucleotide (NAD) and is converted into acetoacetic acid (AcAc). In this case, the amount of resulting reduced nicotinamide adenine dinucleotide (NADH) is measured, for instance, from its absorbance at the wavelength of 340 nm to determine the amount of D-3-hydroxybutyric acid (D-3-OHBA) therefrom. In order to increase accuracy of the absorbancy measurement of the reduced nicotinamide adenine dinucleotide (NADH), hydrazine sulfate is added to the reaction system to cause a reaction thereof with acetoacetic acid (AcAc) for removal of the latter from the system.

Alternatively acetoacetic acid (AcAc) in a body fluid or urine is reacted in the presence of 3-hydroxybutyric dehydrogenase (3-OHBDH) with reduced nicotinamide adenine dinucleotide (NADH) and converted into D-3-hydroxybutyric acid (D-3-OHBA). In this case, amounts of reduced nicotinamide adenine dinucleotide (NADH) present before and after the reaction are respectively measured, for instance, through an absorbancy measurement at the wavelength of 340 nm to determine the reduced amount thereof and the amount of acetoacetic acid therefrom.

For carrying out such measurements, however, a deproteinization, dialysis or the like operation will be required as a pretreatment for selectively and quantitatively determining an amount of D-3-hydroxybutyric acid or acetoacetic acid and requires a dilution of the sample solution (body fluid or urine), which reduces the sensitivity of the measurement. It is assumed that a main cause of requiring such pretreatment lies in preventing interference of lactic dehydrogenase, lactic acid, pyruvic acid and the like present in the sample of body fluid or urine against the reaction referred to.

Therefore, a principal object of the invention is to provide a sensitive method of making diagnosis of diabetes, through an enzymatic quantitative determination of D-3-hydroxybutyric acid or aceotacetic acid in a sample of body fluid or urine, which requires no pretreatment of the sample.

According to the invention, the object is attained by the presence of oxalic or oxamic acid in the determination system.

According to one aspect of the invention, we provide a method of diagnosis of diabetes, which comprises the steps of converting D-3-hydroxybutyric acid in a sample of mammalian body fluid or urine into acetoacetic acid in the presence of nicotinamide adenine dinucleotide and a 3-hydroxybutyric dehydrogenase, colorimetrically measuring the increase in amount of reduced nicotinamide adenine dinucleotide, determining D-3-hydroxybutyric acid based on the increase of reduced nicotinamide adenine dinucleotide, and checking the change of the determined amount of D-3-hydroxybutyric acid, characterised in that oxalic acid is present in the determination system of D-3-hydroxybutyric acid, so that a pretreatment of deproteinization and/or dialysis of the sample is omitted.

According to another aspect of the invention, we provide a method of diagnosis of diabetes, which comprises the steps of converting acetoacetic acid in a sample of mammalian body fluid or urine into D-3-hydroxybutyric acid in the presence of reduced nicotinamide adenine dinucleotide and a 3-hydroxybutyric dehydrogenase, colorimetrically measuring the decrease in an amount of the reduced nicotinamide adenine dinucleotide, determining the acetoacetic acid based on the decrease of reduced nicotinamide adenine dinucleotide, and checking the change of the determined amount of acetoacetic acid, characterised in that oxamic acid is present in the determination system of acetoacetic acid, so that a pretreatment of deproteinization and/or dialysis of the sample is omitted.

The invention also provides reagents for carrying out such determinations, namely (a) nicotinamide adenine dinucleotide, a 3-hydroxybutyric dehydrogenase and oxamic acid; and (b) reduced nicotine adenine dinucleotide, a 3-hydroxybutyric dehydrogenase and oxamic acid.

It is preferable that the oxalic or oxamic acid is present in the measuring system in a concentration of about 56 mg/dl.

The 3-hydroxybutyric dehydrogenase enzyme can be obtained from *Rhodospirillum rubrum*, *Rhodopseudomonas spheroides*, *Pseudomonas lemoignei* and the like bacteria or extracted from a bovine heart, swine kidney and the like animal organs.

Nicotinamide adenine dinucleotide is a coenzyme or activator having a hydrogen atom accepting function in an oxidation-reduction reaction system and can be extracted from a liver, kidney and the like animal organs as well as from yeasts. A sodium or lithium salt thereof may also be employed in the invention.

In either of the methods according to the invention the colorimetry is usually carried out by absorbancy measurement at 340 nm, which is the most basic measure for determining the variation in amount of reduced nicotinamide adenine dinucleotide and thus such absorbancy measurement have been employed in preferable embodiments described below, but it may also be carried out through a fluorescent spectroscopy which directly measures the reduced nicotinamide adenine dinucleotide or utilizes its reduction ability.

Further, each method according to the invention can also be carried out with the aid of a method wherein D-3-hydroxybutyric acid is dehydrogenated through an enzymatic reaction to form acetoacetic acid in the presence of an electron transmitter and a tetrazolium salt which is reduced into a colored formazane which is determined by colorimetry. In this case, it is necessary to add further an electron transmitter and a tetrazolium salt in the measuring system, said tetrazolium salt being reduced to form a coloring formazane to be subjected to a colorimetry. As the electron transmitter, any one can be employed, if it activates a hydrogen atom supplying function of reduced nicotinamide adenine dinucleotide and for instance, diaphorase, 1-methoxy-5-methylphenazium methylsulfate and the like can be used. As the tetrazolium salt, 3-(*p*-iodophenyl)-2-(*p*-nitrophenyl)-5-phenyl-2H tetrazolium chloride or the like monotetrazolium salt and 3,3′-(3,3′-dimethoxy-4,4′-biphenylylene)-bis [2-(*p*-nitrophenyl)-5-phenyl-2H tetrazolium] chloride or the like ditetrazolium salt can be used.

Each of the aforesaid reagents according to the invention and containing the electron transmitter and tetrazolium salt may be soaked up by absorbent carrier such as a filter paper or applied on a support, so that a resulting element can be dried to use the same as a testing member for the colorimetry, by dipping it in the sample.

In the accompanying drawings:

Figures 1 and 2 are graphs showing respectively a change in absorbancy of NADH, for the case when lactic acid exists in a determination system of serum D-3-hydroxybutyric acid or is added therein;

Figures 3 and 4 are graphs showing respectively a change in absorbancy of NADH, for the case when pyruvic acid exists in a determination system of serium acetoacetic acid or is added therein;

Figure 5 is a graph showing a change in absorbancy of NADH, for the case when lactic acid is added in a determination system of urinary D-3-hydroxybutyric acid;

Figure 6 is a graph showing a change in absorbancy of NADH, for the case when pyruvic acid is added in a determination system of urinary acetoacetic acid;

Figures 7 and 8 are graphs showing a comparison of a determination method of azo-compound produced by diazo-reaction, as disclosed in Jap. Pat. Appln. No. 112 520/1982 (Jap. Unexamined Pat. Appln.

Gazette No. 5 959/1984) with that of the present invention, wherein Figure 7 is for D-3-hydroxybutyric acid and Figure 8 is for acetoacetic acid;

Figure 9 is a graph showing an inhibitation by oxamic acid against serum lactic dehydrogenase; and

Figures 10 and 11 show respectively a calibration curve prepared with use of a standard solution of D-3-hydroxybutyric acid or acetoacetic acid and with or without addition of oxamic acid.

The invention will now be further explained in connection with preparations of various agents and execution of various tests.

I. Basic reagents

(1) Coenzyme reagent for quantitative determination of D-3-hydroxybutyric acid

To 23.7 ml of 0.2 mol/l tris chloride buffer (pH 8.5), 3.0 ml of 20 mmol/l NAD and 3.0 ml of 0.5 (w/v)% hydrazine sulfate solution dissolved in the tris chloride buffer were added to stir the resulting solution. To the mixture, 25 mg of oxamic acid was added and dissolved therein and then distilled water was added to make a total volume of the solution to 33.5 ml.

(2) Coenzyme reagent for quantitative determination of acetoacetic acid

To 24.7 ml of 0.25 mol/l triethanolamine solution (pH 7.0), 5.0 ml of 3 mmol/l NADH was added to stir the solution. To the solution, 25 mg of oxamic acid was added and dissolved therein and then distilled water was added to make a total volume of the solution to 33.5 ml.

(3) Enzyme reagent

A solution of 3-hydroxybutyric dehydrogenase, which was prepared by adjusting a commercial one (Grade II) sold by Boehringer Mannheim GmbH, West Germany to about 15.0 U/ml (25°C).

II. Procedure of manual method

(A) Quantitative determination of D-3-hydroxybutyric acid:

A test solution was prepared by firstly mixing the coenzyme reagent (1) with the enzyme reagent (3) in a ratio of 67:3 to prepare a mixed reagent solution and secondly adding 300 µl of a sample solution (a body fluid or urine) to 1050 µl of the mixed reagent solution to mix well. The absorbancy (Ax) of the test solution was measured at 340 nm, immediately. Then the test solution was shaken for 5 minutes at 37°C to incubate the same and the absorbancy (Ay) of the incubated test solution was also measured at 340 nm. In a similar manner, each of absorbancies (Bx, By and Sx, Sy) of a blank solution and a standard solution was measured at the time before and after an incubation reaction at 37°C, each of said blanks being distilled water and said standard solutions being prepared by dissolving 500 µmol/l sodium D-3-hydroxybutyrate in 6% albumin solution, instead of the sample solution.

A content (Ea) of D-3-hydroxybutyric acid in the sample solution was calculated by the following equation:

$$Ea\ (\mu mol/l) = \frac{Ay-Ax-(By-Bx)}{Sy-Sx-(By-Bx)} \times 500$$

In case of adding the enzyme and coenzyme reagents separately, such operation manner is modified as follows. To 1005 µl of the coenzyme reagent (1) for quantitative determination of D-3-hydroxybutyric acid, 300 µl of the sample solution was added to measure an absorbancy (Ax') thereof and then 45 µl of the enzyme reagent (3) was added thereto to shake the resulting solution for 5 minutes at 37°C and to measure an absorbancy thereof. Absorbancies of blank and standard solutions were measured in a manner similar thereto. On calculation in this case, the following figures should be employed instead of Ax, Bx and Sx, respectively.

$$Ax' \times \frac{1050}{1005},\ Bx' \times \frac{1050}{1005},\ Sx' \times \frac{1050}{1005}$$

(B) Quantitative determination of acetoacetic acid:

A test solution was prepared by firstly mixing the coenzyme reagent (2) with the enzyme reagent (3) in a ratio of 67:3 to prepare a mixed reagent solution and secondary adding 300 µl of a sample solution to 1050 µl of the mixed reagent solution to mix well. The absorbancy (Cx) of the test solution was measured at 340 nm, immediately. Then the test solution was shaken for 5 minutes at 37°C to incubate the same and an absorbancy (Cy) of the incubated test solution was also measured at 340 nm. In a similar manner, each of absorbancies (B'x, B'y and S'x, S'y) of a blank solution and a standard solution was measured at the time before and after an incubate reaction at 37°C, each of said blank being distilled water and said standard solutions being prepared by dissolving 500 µmol/l lithium acetoacetate in 6% albumin solution, instead of the sample solution.

A content (Eb) of acetoacetic acid in the sample solution was calculated by following equation.

$$Eb\ (\mu mol/l) = \frac{Cx\text{-}Cy\text{-}(B'x\text{-}B'y)}{S'x\text{-}S'y\text{-}(B'x\text{-}B'y)} \times 500$$

In case of adding the enzyme and coenzyme reagents separately, such operation manner is modified as follows. To 1005 µl of the coenzyme reagent (2) for quantitative determination of acetoacetic acid, 300 µl of the sample solution was added to measure an absorbancy (Cx') thereof and then 45 µl of the enzyme reagent (3) was added thereto to shake the resulting solution for 5 minutes at 37°C and to measure an absorbancy thereof. Absorbancies of blank and standard solutions were measured in a similar manner. On calculation in this case, following figures should be employed instead of Cx, B'x and S'x, respectively.

$$Cx' \times \frac{1050}{1005},\ B'x' \times \frac{1050}{1005},\ S'x' \times \frac{1050}{1005}$$

Figure 1 shows a change in absorbancy of NADH at 340 nm, when lactic acid exists in a determination system for D-3-hydroxybutyric acid in a sample solution of blood serum and Figure 2 shows a similar change when lactic acid was added to the system.

In the Figures, Curves 1, 2 and 3 show changes in absorbancy in case of adding a 3-hydroxybutyric dehydrogenase without addition of oxamic acid, adding neither the 3-hydroxybutyric dehydrogenase nor oxamic acid, and adding both of the substances, respectively.

It can be seen from the Figures that the lactic acid existing in the system causes the change in absorbancy of NADH at 340 nm but such change can almost completely be inhibited by oxamic acid as a lactic dehydrogenase inhibitor.

While, Figure 3 shows a change in absorbancy of NADH at 340 nm, when a small amount of pyruvic acid exists in a determination system for acetoacetic acid in a sample solution of blood serum and Figure 4 shows a similar change when pyruvic acid was added to the system.

In the Figures 3 and 4, Curves 1, 2 and 3 show changes in absorbancy, as similar to those in Figures 1 and 2. It can be seen from Figures 3 and 4 that the pyruvic acid existing in the system causes the change in absorbancy of NADH at 340 nm but such change can almost completely be inhibited by oxamic acid as a lactic dehydrogenase inhibitor.

In case of that lactic acid was added in the system for D-3-hydroxybutyric acid in a urinary sample, a change in absorbancy of NADH at 340 nm was checked and shown in Figure 5. In case of that pyruvic acid was added in the system for acetoacetic acid in a urinary sample, a change in absorbancy of NADH at 340 nm was also checked and shown in Figure 6. In the Figures 5 and 6, Curves 1, 2 and 3 show changes in absorbancy as in Figure 1. An inhibitation effect by oxamic acid was not clearly seen from Figure 5, since an enzymatic activity of the lactic dehydrogenase in urine is lower than that in blood serum and an interference of lactic acid in urine is lower than that in blood serum. However, it can be seen from Figure 6 that the change in absorbancy of NADH by pyruvic acid can almost completely be inhibited by oxamic acid as a lactic dehydrogenase inhibitor.

III. Procedure utilizing a centrifugal autoanalyzer (analyzer: "Centrifichem System 500" by Richardson Vicks Corp., U.S.A.)

(A) Quantitative determination of D-3-hydroxybutyric acid:

100 µl of a sample solution, 100 µl of 500 µmol/l sodium D-3-hydroxybutyrate standard solution prepared with use of 6% albumin solution and 100 µl of distilled water as a blank were accurately pipetted into sample cavities on a transferdisk of the analyzer, respectively. Each 350 µl of a test solution prepared by mixing the coenzyme reagent (1) for quantitative determination of D-3-hydroxybutyric acid and the enzyme reagent (3) in a ratio of 67:3 was pipetted with use of System 500 pipetter into reagent cavities on the transferdisk of the analyzer. Thereafter, the transferdisk was set in a rotor in its chamber of the analyzer, conditions for which had previously been set as shown in following Table 1, to start measurements.

TABLE 1

| | |
|---|---|
| Temperature | 37°C |
| Filter | 340 nm |
| To | 3 sec. |
| T | 6 min. |
| Blank | Auto |
| Test mode | Term |
| Standard | 500 µmol/l |
| No. of print | 1 |

(B) Quantitative determination of acetoacetic acid

100 µl of a sample solution, 100 µl of 500 µmol/l lithium acetoacetate standard solution prepared with use of 6% albumin solution and 100 µl of distilled water as a blank were accurately pipetted into sample cavities on a transferdisk of the analyzer, respectively. Each 350 µl of a test solution prepared by mixing the coenzyme reagent (2) for quantitative determination of acetoacetic acid and the enzyme reagent (3) in a ratio of 67:3 was pipetted with use of System 500 pipetter into a reagent cavity on the transferdisk of the analyzer. Thereafter, the transferdisk was set in a rotor chamber of the analyzer, conditions for which had previously been set as shown in said Table 1, to start measurements.

According to the procedures as referred to, an increase or decrease in absorbancy of NADH at 340 nm can be measured through end point method to directly and quantitatively determine D-3-hydroxybutyric acid or acetoacetic acid in a body fluid or urine, without requiring any pretreatment of deproteinization or dialysis. Linearities were confirmed up to 800 µmol/l for D-3-hydroxybutyric acid and 1000 µmol/l for acetoacetic acid. An intra-assay variance was confirmed as CV 2—4%, and a recovering ratio of D-3-hydroxybutyric acid or acetoacetic acid added in blood serum or urine was 98—108% in each of the measuring system.

A correlation of this method with the previously offered method as disclosed in Jap. Pat. Appln. No. 112 520/1982 (Jap. Unexamined Pat. Appln. Gazette No. 5 959/1984) which employs a colorimetric assay of azo-compounds produced by a diazo-reaction was checked to obtain results as shown in Figures 7 and 8. The both methods have a good correlation of that a correlation coefficient $r$ is 0.99 for D-3-hydroxybutyric acid and 0.97 for acetoacetic acid. The results clearly show a fact that the presence of the lactic dehydrogenase inhibitor makes a pretreatment such as deproteinization or dialysis unnecessary.

A lactic dehydrogenase inhibitation coefficient in blood serum by oxamic acid was studied to obtain a result as shown in Figure 9. The concentration of oxamic acid used in this study was 56 mg/dl, which inhibits lactic dehydrogenase of 95% or more.

Further, an influence of oxamic acid on absorbancy of NADH at 340 nm was checked through experiments utilizing a standard calibration curve to obtain results as shown in Figures 10 and 11. As seen from the Figures, there is no notifiable influence.

When a pipetter for the analyzer (System 500) is employed, a sample volume and a total volume should be set into 45 µl and 95 µl, respectively, which makes its linearity about 2 times, although sensitivity thereof shall be reduced into about an half.

According to the procedures, quantitative determinations of serum acetoacetic acid and D-3-hydroxy butyric acid were separately carried out on 20 healthy humans, results thereof being shown in following Table 2 in a mean value ±S.D (standard deviation) (µmol/l). Acetoacetic acid and D-3-hydroxybutyric acid in patient sera with diabetes, one of which was ketoacidosis, were separately and quantitatively determined, and results thereof are shown in the Table 3 below in a mean value ±S.D (µmol/l). The following Table 4 shows results of separate quantitative determination on 5 urine samples, two of which showed a positive reaction in a determination by nitroprusside method.

TABLE 2

| Sample | µmol/l | |
|---|---|---|
| | Acetoacetic acid | D-3-hydroxy-butyric acid |
| Sera from 20 healthy humans | 43.8±14.05 | 40.6±17.15 |

TABLE 3

| Sample No. | µmol/l | |
|---|---|---|
| | Acetoacetic acid | D-3-hydroxy-butyric acid |
| 1 | 259 | 570 |
| 2 | 175 | 532 |
| 3 | 163 | 646 |
| 4 | 339 | 785 |
| 5 | 1284 | 1660 |
| 6 | 59 | 121 |
| 7 | 254 | 724 |
| 8 | 279 | 577 |
| 9 | 115 | 264 |
| 10 | 96 | 352 |
| mean value ±S.D* | 193.2±94.51 | 507.9±219.45 |

*:value on 9 samples excluding No. 5 sample

TABLE 4

| Sample No. | Method of the invention (µmol/l) | | Nitroprusside method |
|---|---|---|---|
| | Acetoacetic acid | D-3-hydroxy-butyric acid | |
| 1 | 9 | 13 | – |
| 2 | 1 | 1 | – |
| 3 | 1082 | 402 | ++ |
| 4 | 1114 | 437 | ++ |
| 5 | 24 | 17 | – |

**Claims**

1. A method of diagnosis of diabetes, which comprises the steps of converting D-3-hydroxybutyric acid

in a sample of mammalian body fluid or urine into acetoacetic acid in the presence of nicotinamide adenine dinucleotide and a 3-hydroxybutyric dehydrogenase, colorimetrically measuring the increase in amount of reduced nicotinamide adenine dinucleotide, determining D-3-hydroxybutyric acid based on the increase of reduced nicotinamide adenine dinucleotide, and checking the change of the determined amount of D-3-hydroxybutyric acid, characterized in that oxalic acid is present in the determination system of D-hydroxybutyric acid, so that a pretreatment of deproteinization and/or dialysis of the sample is omitted.

2. A method of diagnosis of diabetes, which comprises the steps of converting acetoacetic acid in a sample of mammalian body fluid or urine into D-3-hydroxybutyric acid in the presence of reduced nicotinamide adenine dinucleotide and a 3-hydroxybutyric dehydrogenase, colorimetrically measuring the decrease in amount of the reduced nicotinamide adenine dinucleotide, determining the acetoacetic acid based on the decrease of reduced nicotinamide adenine dinucleotide, and checking the change of the determined amount of acetoacetic acid, characterized in that oxamic acid is present in the determination system of acetoacetic acid, so that a pretreatment of deproteinization and/or dialysis of the sample is omitted.

3. A reagent for carrying out the method as claimed in Claim 1, which comprises nicotinamide adenine dinucleotide, a 3-hydroxybutyric dehydrogenase and oxalic acid.

4. A reagent for carrying out the method as claimed in Claim 2, which comprises reduced nicotinamide adenine dinucleotide, a 3-hydroxybutyric dehydrogenase and oxamic acid.

**Patentansprüche**

1. Methode zur Diagnose von Diabetis, die folgende Stufen umfasst: das Umwandeln der D-3-Hydroxy-Buttersäure in einer Probe von Körperflüssigkeit oder Urin von Säugetieren in Acetessigsäure in Anwesenheit von Nicotinamid-Adenin-Dinucleotid und einer 3-Hydroxybutter Dehydrogenase, das colorimetrische Messen des Ansteigens der Menge von reduziertem Nicotinamid-Adenin-Dinucleotid, die Bestimmung der D-3-Hydroxy-Buttersäure auf der Grundlage des Anstiegs des reduzierten Nicotinamid-Adenin-Dinucleotid und das Ueberprüfen des Wechsels der bestimmten Menge von D-3-Hydroxy-Buttersäure, dadurch gekennzeichnet, dass Oxalsäure im Bestimmungssystem der D-3-Hydroxy-Buttersäure vorhanden ist, so dass eine Deproteinisierungs- und/oder Dialysevorbehandlung der Probe entfällt.

2. Methode zur Diagnose von Diabetis, die folgende Stufen umfasst: das Umwandeln der Acetessigsäure in einer Probe von Körperflüssigkeit oder Urin von Säugetieren in D-3-Hydroxy-Buttersäure unter Mitwirkung von reduziertem Nicotinamid-Adenin-Dinucleotid und einer 3-Hydroxybutter Dehydrogenase, das colorimetrische Messen der Verringerung der Menge an reduziertem Nicotinamid-Adenin-Dinucleotid, das Bestimmen der Acetessigsäure auf der Grundlage der Verringerung des reduzierten Nicotinamid-Adenin-Dinucleotid und das Ueberprüfen des Wechsels der bestimmten Menge von Acetessigsäure, dadurch gekennzeichnet, dass die Oxaminsäure im Bestimmungssystem für die Acetessigsäure vorhanden ist, so dass eine Deproteinisierungs- und/oder Dialysevorbehandlung der Probe entfällt.

3. Reagenz zum Durchführen der Method nach Anspruch 1, das Nicotinamid-Adenin-Dinucleotid, 3-Hydroxybutter Dehydrogenase und Oxalsäure umfasst.

4. Reagenz zum Durchführen der Method nach Anspruch 2, das reduziertes Nicotinamid-Adenin-Dinucleotid, eine 3-Hydroxybutter Dehydrogenase und Oxaminsäure umfasst.

**Revendications**

1. Procédé pour diagnostiquer le diabète qui, comprend les stades de convertir l'acide D-3-hydroxybutyrique dans un échantillon d'urine ou de liquide de l'organisme d'un mammifère en acide acétoacétique en présence de nicotinamide-adénine-dinucléotide et d'une déshydrogénase 3-hydroxybutyrique, de mesurer par colorimétrie l'augmentation de la quantité de nicotinamide-adénine-dinucléotide réduit, de déterminer l'acide D-3-hydroxybutyrique sur base de l'augmentation du nicotinamide-adénine-dinucléotide réduit, et de vérifier la modification de la quantité déterminée d'acide D-3-hydroxybutyrique, caractérisé en ce que l'acide oxalique est présent dans le système de détermination de l'acide D-3-hydroxybutyrique, de façon qu'un traitement préalable d'élimination des protéines et/ou de dialyse de l'échantillon soit omis.

2. Procédé pour diagnostiquer le diabète, qui comprend les stades de convertir l'acide acétoacétique dans un échantillon d'urine ou de liquide de l'organisme d'un mammifère en acide D-3-hydroxybutyrique en présence de nicotinamide-adénine-dinucléotide réduit et d'une déshydrogénase 3-hydroxybutyrique, de mesurer par colorimétrie la diminuation de la quantité de nicotinamide-adénine-dinucléotide réduit, et de vérifier la modification de la quantité déterminée d'acide acétoacétique, caractérisé en ce que l'acide oxamique est présent dans le système de détermination de l'acide acétoacétique, de façon qu'un traitement préalable d'élimination des protéines et/ou de dialyse de l'échantillon soit omis.

3. Réactif pour exécuter le procédé suivant la revendication 1, qui comprend du nicotinamide-adénine-dinucléotide, une déshydrogénase 3-hydroxybutyrique et de l'acide oxalique.

4. Réactif pour exécuter le procédé suivant la revendication 2, qui comprend du nicotinamide-adénine-dinucléotide réduit, une déshydrogénase 3-hydroxybutyrique et de l'acide oxamique.

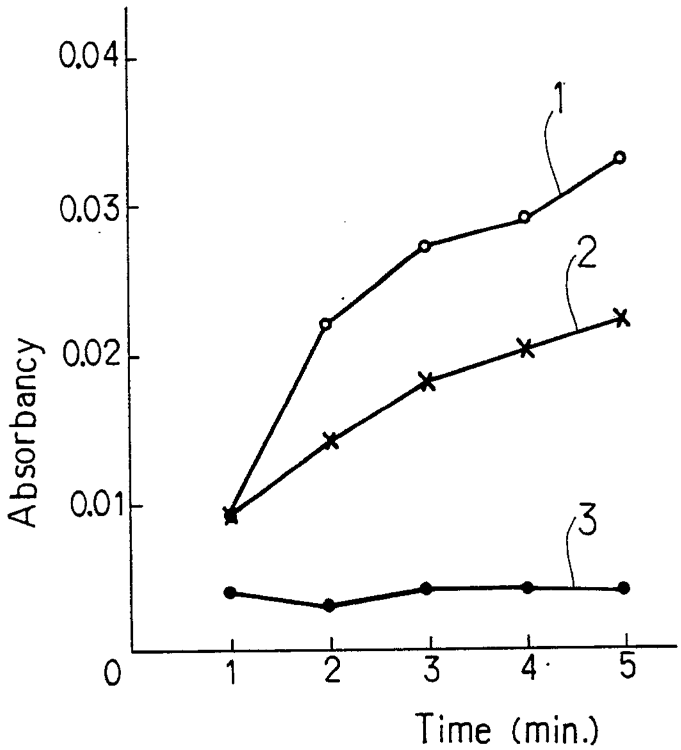
0.04
0.03
0.02
0.01
Absorbancy
0
1
2
3
4
5
Time (min.)
1
2
3

FIG. 1

FIG. 2

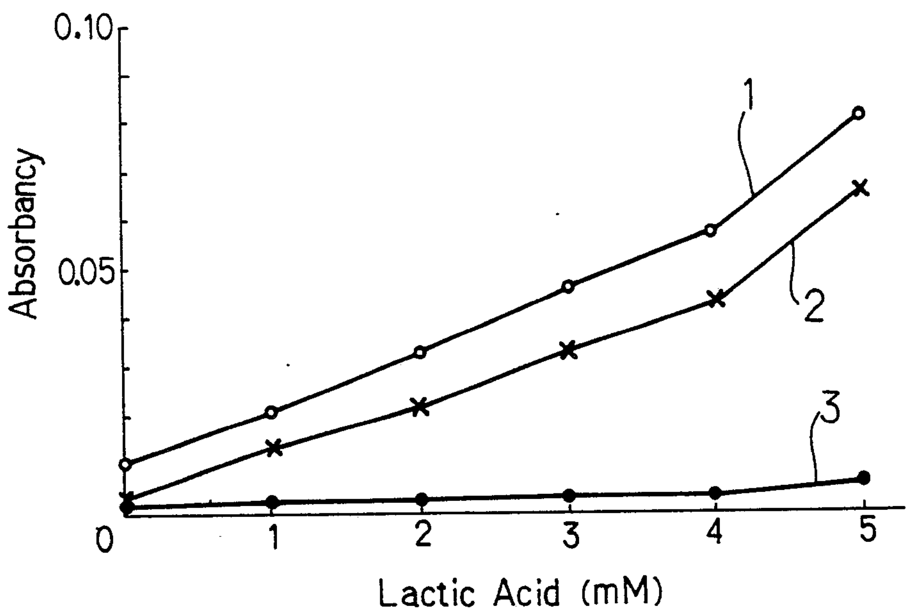
0.10
0.05
Absorbancy
0
1
2
3
4
5
Lactic Acid (mM)
1
2
3

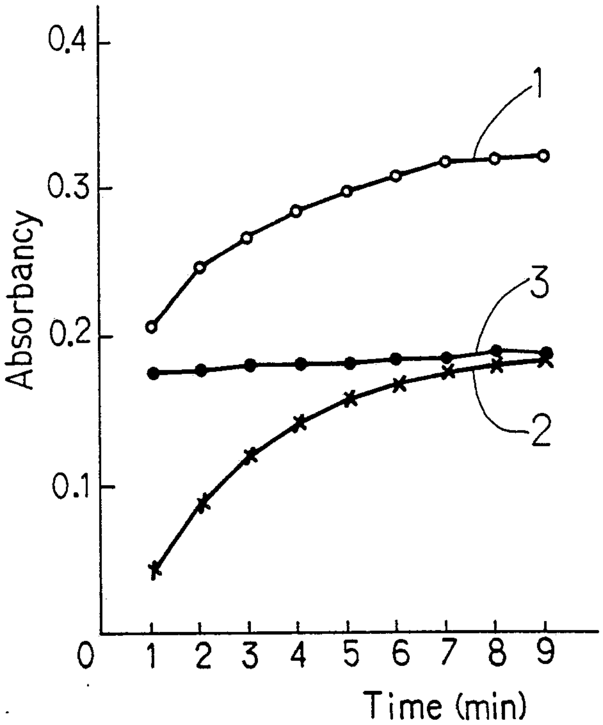
0.4
0.3
0.2
0.1
Absorbancy
1
3
2
0
1 2 3 4 5 6 7 8 9
Time (min)

FIG.3

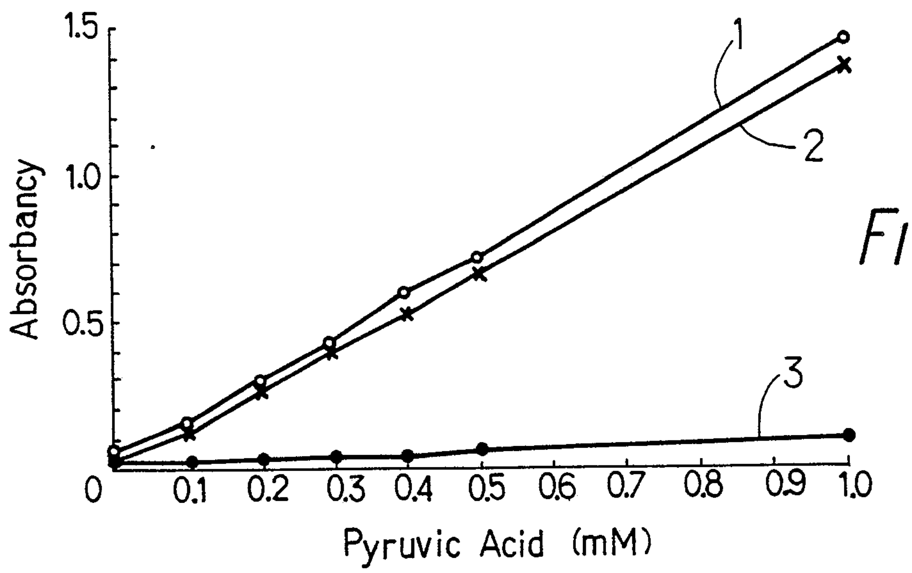
1.5
1.0
0.5
Absorbancy
1
2
3
0
0.1 0.2 0.3 0.4 0.5 0.6 0.7 0.8 0.9 1.0
Pyruvic Acid (mM)

FIG.4

FIG. 5

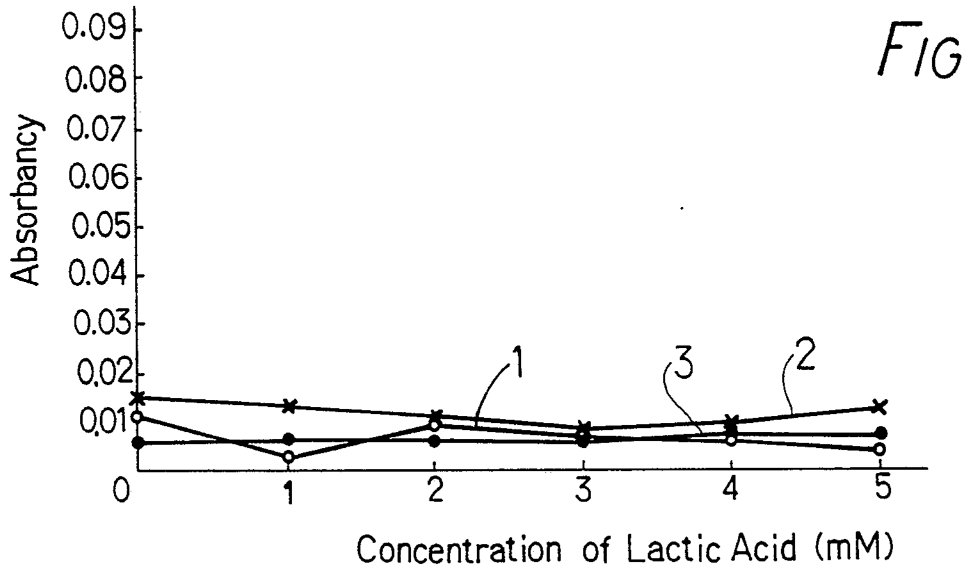
Absorbancy
0.09
0.08
0.07
0.06
0.05
0.04
0.03
0.02
0.01
0
1
2
3
4
5
1
3
2
Concentration of Lactic Acid (mM)

FIG. 6

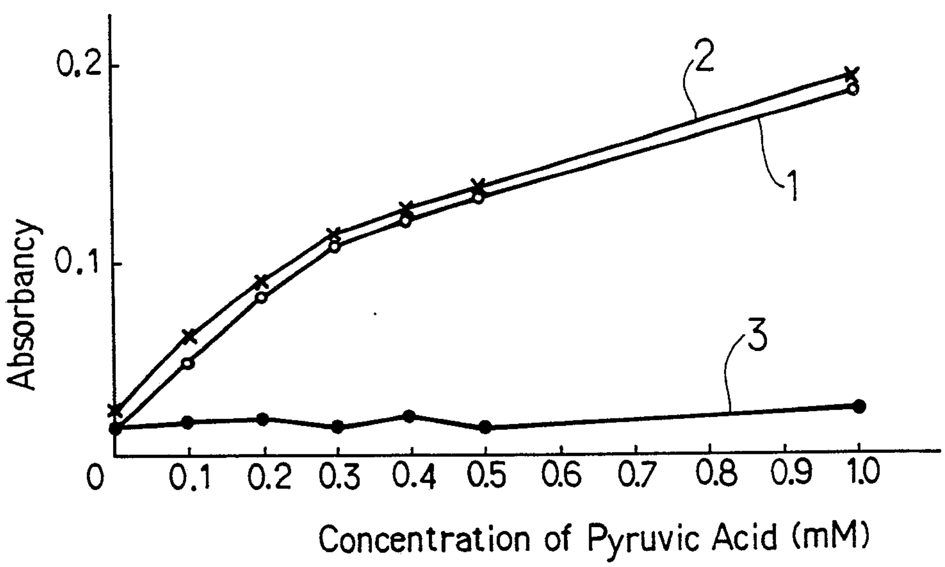
Absorbancy
0.2
0.1
0
0.1
0.2
0.3
0.4
0.5
0.6
0.7
0.8
0.9
1.0
2
1
3
Concentration of Pyruvic Acid (mM)

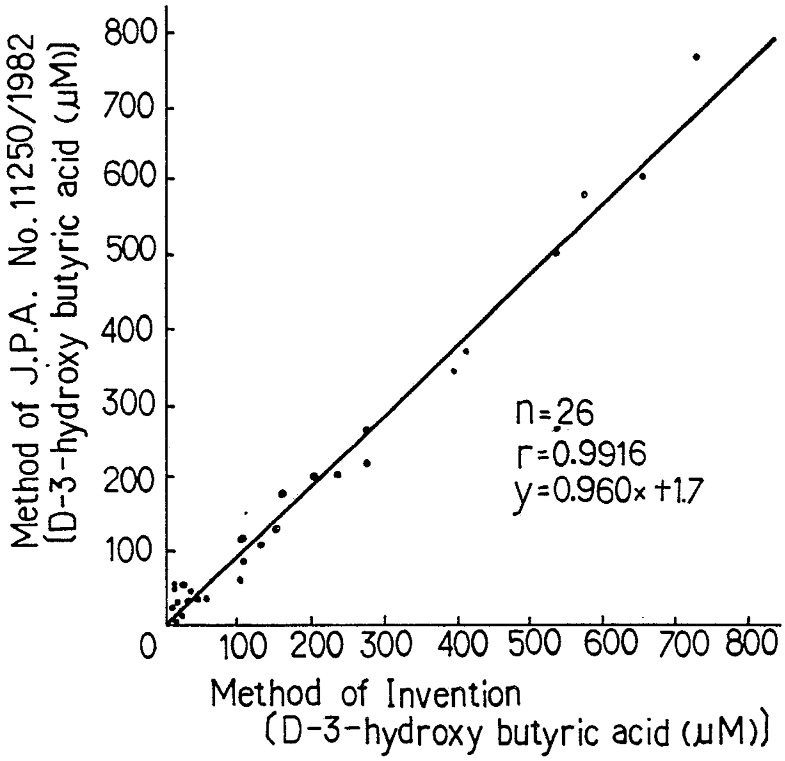
Method of J.P.A. No.11250/1982
(D-3-hydroxy butyric acid (μM))
800
700
600
500
400
300
200
100
0
100 200 300 400 500 600 700 800
Method of Invention
(D-3-hydroxy butyric acid (μM))
n=26
r=0.9916
y=0.960x+1.7

FIG.7

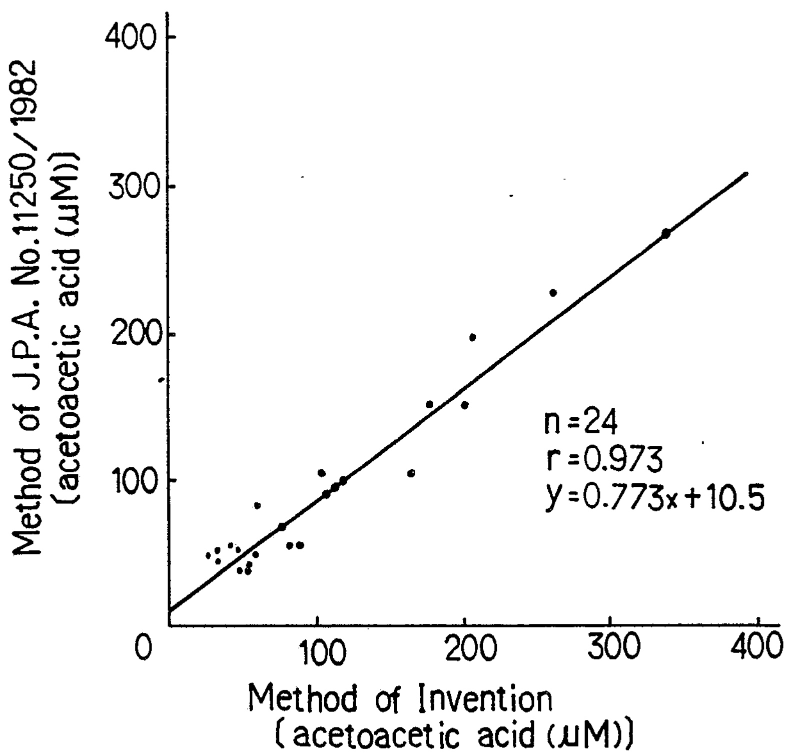
Method of J.P.A. No.11250/1982
(acetoacetic acid (μM))
400
300
200
100
0
100 200 300 400
Method of Invention
(acetoacetic acid (μM))
n=24
r=0.973
y=0.773x+10.5

FIG.8

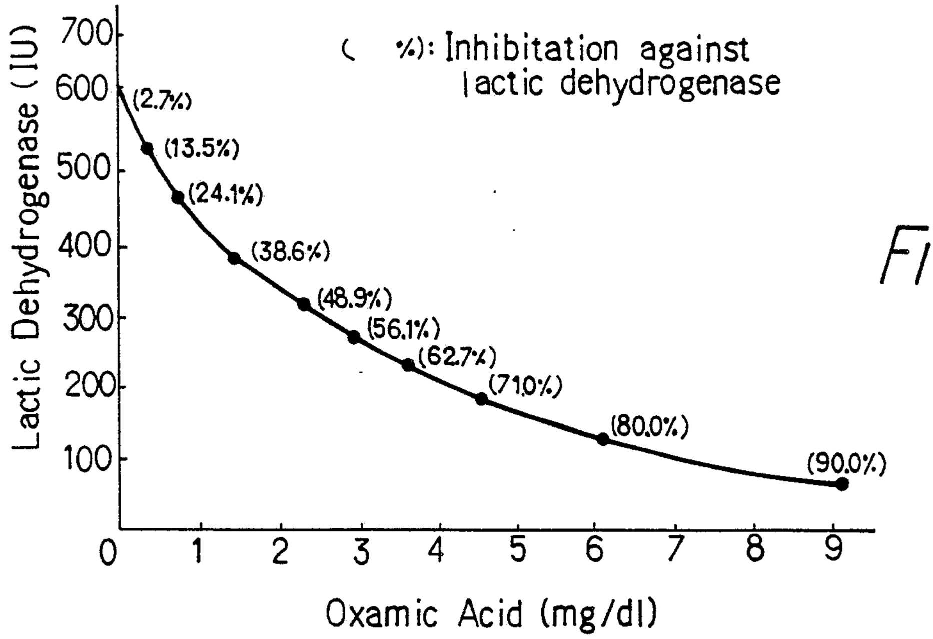
( %): Inhibitation against lactic dehydrogenase
Lactic Dehydrogenase (IU)
700
600
500
400
300
200
100
(2.7%)
(13.5%)
(24.1%)
(38.6%)
(48.9%)
(56.1%)
(62.7%)
(71.0%)
(80.0%)
(90.0%)
0
1
2
3
4
5
6
7
8
9
Oxamic Acid (mg/dl)

FIG.9

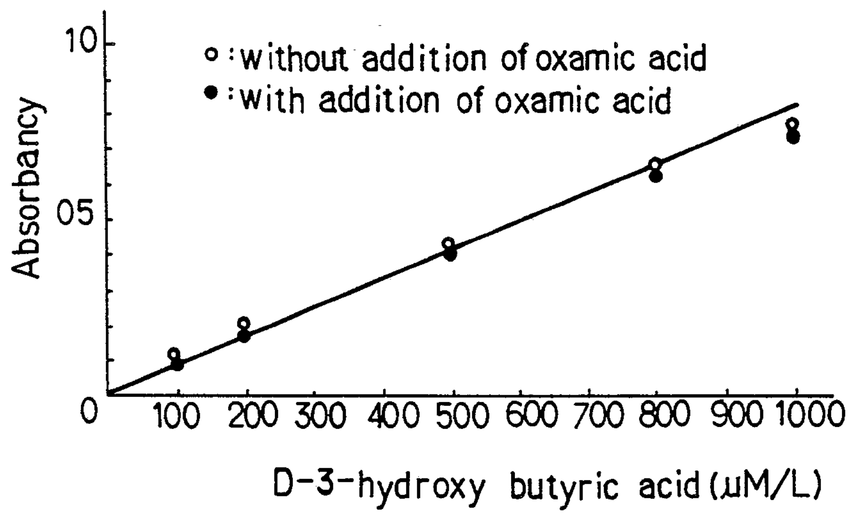
○ : without addition of oxamic acid
● : with addition of oxamic acid
Absorbancy
10
05
0
100
200
300
400
500
600
700
800
900
1000
D-3-hydroxy butyric acid (µM/L)

FIG.10

FIG.11

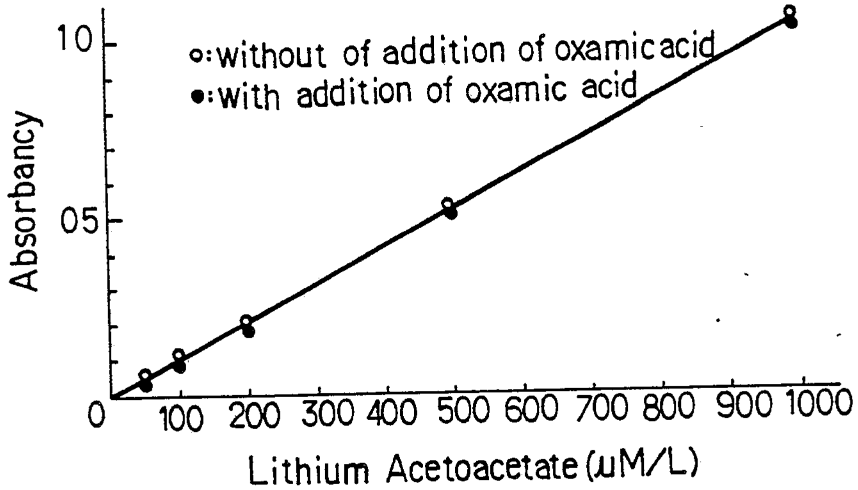

10
05
0
Absorbancy
○:without of addition of oxamic acid
●:with addition of oxamic acid
100 200 300 400 500 600 700 800 900 1000
Lithium Acetoacetate (μM/L)